# EUROPÄISCHE PATENTSCHRIFT

(11) **EP 0 384 310 B1**
(45) Veröffentlichungstag und Bekanntmachung des Hinweises auf die Patenterteilung: **06.07.1994**
(21) Anmeldenummer: 90103016.3
(22) Anmeldetag: 16.02.1990
(51) Int. Cl.: C07D 271/06

(54) **Verfahren zur Herstellung von Phenyloxdiazolylanilinen**
Process for the preparation of phenyl oxadiazolyl anilines
Procédé pour la préparation de phényl-oxadiazolyl-anilines

(30) Priorität: 21.02.1989 DE 3905242
(43) Veröffentlichungstag der Anmeldung: 29.08.1990
(73) Patentinhaber: BASF Aktiengesellschaft, 67063 Ludwigshafen (DE)
(72) Erfinder: Jesse, Joachim, Dr., D-6714 Weisenheim (DE); Kanter, Hartmut, Dr., D-6700 Ludwigshafen (DE)

(56) Entgegenhaltungen:
- DE-A- 2 101 559
- CHEMICAL PHARMACEUTICAL BULLETIN, Band 23, Nr. 12, Dezember 1975, Seiten 3178-3183, Tokyo, JP; K. NAGAHARA et al.: "Sur la formation d'oxadiazoles-1,2,4 par action de benzamidoximes sur des anhydrides isatoiques"

## Beschreibung

Die vorliegende Erfindung betrifft ein neues Verfahren zur Herstellung von Phenyloxdiazolylanilinen durch Umsetzung von einem Benzonitril in wäßrigem Reaktionsmedium zunächst mit Hydroxylamin oder dessen Säureadditionssalzen zu einem Benzamidoxim und anschließend mit einem Isatosäureanhydrid zu einer ringoffenen Verbindung und nachfolgender Cyclisierung, wobei man die Umsetzung in Gegenwart eines anionischen Tensids durchführt.

Aus der DE-A-2 457 687 ist bereits die Herstellung von 2-(3-Phenyl-1,2,4-oxdiazol-5-yl)anilin bekannt. Nach der dort beschriebenen Methode wird Benzonitril zunächst in einem Gemisch aus Wasser und Isobutanol mit Hydroxylammoniumsulfat in Gegenwart von Natriumcarbonat zu Benzamidoxim umgesetzt. Das Benzamidoxim läßt man dann, nach Abtrennung der wäßrigen Phase, in der Isobutanolphase mit Isatosäureanhydrid in Gegenwart von feingepulvertem Natriummethanolat zum Zielprodukt weiterreagieren.

Das so erhaltene Phenyloxdiazolylanilin dient als Diazokomponente für die in der DE-A-2 457 687 beschriebenen Pigmente. Die Diazotierung des Phenyloxdiazolylanilins mit Natriumnitrit gelingt wegen der geringen Löslichkeit sowohl des Amins wie auch seines Diazoniumsalzes in verdünnter Salzsäure nur unvollständig, was zu einer Beeinträchtigung der anwendungstechnischen Eigenschaften der obengenannten Pigmente führt. Aus diesem Grund muß die Diazotierung von Phenyloxdiazolylanilin in einem Gemisch aus Schwefelsäure und Nitrosylschwefelsäure durchgeführt werden.

Auch wenn man die Umsetzung des Benzamidoxims zu Phenyloxdiazolylanilin nicht in Isobutanol sondern in einem Gemisch aus Isobutanol und Wasser und in Gegenwart von Natronlauge vornimmt, erhält man nach Entfernen des Isobutanols mittels Wasserdampfdestillation ein Phenyloxdiazolylanilin, das sich ebenfalls nicht vollständig in verdünnter Salzsäure diazotieren läßt.

Aufgabe der vorliegenden Erfindung war es nun, ein neues Verfahren bereitzustellen, mittels dessen die Herstellung von Phenyloxdiazolylanilinen in einfacher Weise und in guten Ausbeuten gelingen sollte, wobei die Zielprodukte außerdem gut in verdünnter Salzsäure diazotierbar sein sollten.

Demgemäß wurde gefunden, daß die Herstellung von Phenyloxdiazolylanilinen der Formel I 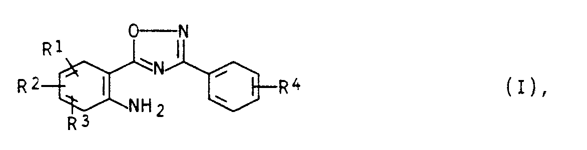
in der
- R¹: Wasserstoff, Halogen, C₁-C₄-Alkyl, C₁-C₄-Alkoxy, Trifluormethyl oder Nitro,
- R²: Wasserstoff, Halogen oder Nitro,
- R³: Wasserstoff oder Halogen und
- R⁴: Wasserstoff, Halogen, C₁-C₄-Alkyl, C₁-C₄-Alkoxy oder Nitro bedeuten,
durch Umsetzung eines Benzonitrils der Formel II 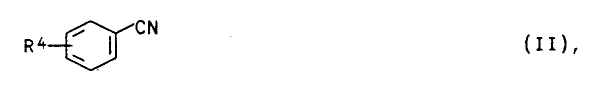
in der R⁴ die obengenannte Bedeutung besitzt, in wäßrigem Reaktionsmedium zunächst mit Hydroxylamin oder dessen Säureadditionssalzen zu einem Benzamidoxim der Formel III 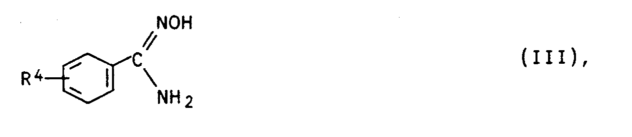
in der R⁴ die obengenannte Bedeutung besitzt, und anschließend mit einem Isatosäureanhydrid der Formel IV 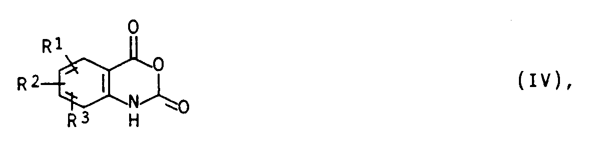
in der R¹, R² und R³ jeweils die obengenannte Bedeutung besitzen, zu einer ringoffenen Verbindung der Formel V 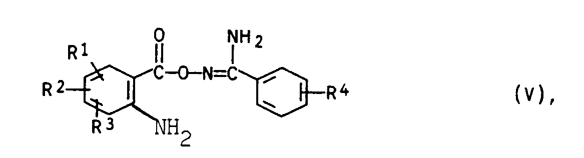
in der R¹, R², R³ und R⁴ jeweils die obengenannte Bedeutung besitzen, die dann cyclisiert wird, vorteilhaft gelingt, wenn man die Umsetzung in Gegenwart eines anionischen Tensids vornimmt.

Alle in der obengenannten Formel I auftretenden Alkylgruppen können sowohl geradkettig als auch verzweigt sein.

R¹, R², R³ und R⁴ sind z.B. Fluor, Chlor oder Brom.

R¹ und R⁴ sind weiterhin z.B. Methyl, Ethyl, Propyl, Isopropyl, Butyl, Isobutyl, sec-Butyl, tert-Butyl, Methoxy, Ethoxy, Propoxy, Isopropoxy, Butoxy, Isobutoxy oder sec-Butoxy.

Im erfindungsgemäßen Verfahren wird das Benzonitril II mit Hydroxylamin oder dessen Säureadditionssalzen umgesetzt. Säureadditionssalze von Hydroxylamin sind z.B. Hydroxylammoniumchlorid, -bromid oder -sulfat.

Vorzugsweise verwendet man ein Säureadditionssalz, wobei Hydroxylammoniumsulfat besonders bevorzugt ist.

Bei den im erfindungsgemäßen Verfahren zur Anwendung kommenden anionischen Tensiden handelt es sich um an sich bekannte Stoffe, wie sie z.B. in Ullmanns Encyklopädie der Technischen Chemie, 4. Auflage, Band 22, Seite 468 bis 488, beschrieben sind.

Beispielhaft seien Carboxylate, wie carboxymethylierte Oxethylate oder Derivate von Aminosäuren, Sulfonate, wie Alkylbenzolsulfonate, Alkylnaphthalinsulfonate, Alkylsulfonate, Olefinsulfonate, Sulfofettsäureester, Sulfofettsäureamide, Sulfobernsteinsäureester, Alkoxyalkylsulfonate, Acyloxyalkylsulfonate oder Acylaminoalkylsulfonate, Sulfate, wie Alkylsulfate oder Ethersulfate, Phosphonate oder Phopshate zu nennen.

Bevorzugt verwendet man anionische Tenside auf Basis von Sulfonaten im erfindungsgemäßen Verfahren. Die Verwendung von Sulfofettsäureestern oder -amiden ist besonders hervorzuheben.

Im allgemeinen verwendet man die anionischen Tenside in einer Menge von 3 bis 20 Gew.%, vorzugsweise 5 bis 10 Gew.%, jeweils bezogen auf das Gewicht an Benzonitril II.

Das neue Verfahren wird in wäßrigen Reaktionsmedium durchgeführt. Vorzugsweise führt man dabei die Umsetzung in Wasser durch, das im wesentlichen frei ist von organischen Lösungsmitteln.

Es ist natürlich auch möglich, die Umsetzung in einem Reaktionsmedium, das zwar überwiegend aus Wasser besteht, aber auch noch in geringem Maße (z.B. bis zu ca. 10 Gew.%, bezogen auf das Gewicht des vorhandenen Wassers) organische Lösungsmittel, z.B. Alkohole, wie Methanol, Ethanol, Propanol, Isopropanol, Butanol oder Isobutanol, enthält, vorzunehmen.

Das erfindungsgemäße Verfahren wird in der Regel so durchgeführt, daß man das Benzonitril der Formel II zusammen mit dem anionischen Tensid in wäßrigem Reaktionsmedium vorlegt und zunächst 1 bis 2 Stunden bei einer Temperatur von 10 bis 40°C dispergiert. Danach gibt man Hydroxylamin oder dessen Säureadditionssalze und eine Base (z.B. Natriumcarbonat, Kaliumcarbonat oder Magnesiumcarbonat) zu und erwärmt das Reaktionsgemisch für 3 bis 5 Stunden auf eine Temperatur von 85 bis 100°C.

Danach kühlt man das Reaktionsgemisch auf eine Temperatur von 55 bis 65°C ab und gibt zum darin befindlichen Benzamidoxim der Formel III Isatosäureanhydrid der Formel IV portionsweise zu, wobei sich zunächst die ringoffene Verbindung V ausbildet.

Durch Zugabe einer starken Base (z.B. Natrium- oder Kaliumhydroxid oder deren wäßrige Lösungen) wird die ringoffene Verbindung V dann bei einer Temperatur von 90 bis 100°C und bei einem pH-Wert von 10 bis 11 zum Oxdiazol cyclisiert. Das Zielprodukt wird anschließend abgesaugt, mit Wasser gewaschen und getrocknet.

Das Molverhältnis von Benzonitril II : Hydroxylamin : Isatosäureanhydrid IV beträgt im allgemeinen 0,9:1:0,8 bis 1:1:1,2.

Die Menge an Base, die man für die Herstellung des Benzamidoxims III benötigt, ist im allgemeinen abhängig von der Art des verwendeten Hydroxylammoniumsalzes und beträgt z.B. bei 1 Val Hydroxylammoniumsulfat ca. 1,0 bis 1,1 Val.

Vorzugsweise führt man das erfindungsgemäße Verfahren mit einem Benzonitril der Formel II, in der R⁴ Wasserstoff bedeutet, und einem Isatosäureanhydrid der Formel IV, in der R¹ Wasserstoff, Chlor oder Brom, R² Wasserstoff, Chlor oder Brom und R³ Wasserstoff bedeuten, durch und gelangt so zu Phenyloxdiazolylanilinen der Formel I, in der R¹ für Wasserstoff, Chlor oder Brom, R² für Wasserstoff, Chlor oder Brom und R³ und R⁴ jeweils für Wasserstoff stehen.

In manchen Fällen kann es zweckmäßig sein, die ringoffene Verbindung V zwischenzuisolieren (z.B. durch Abnutschen oder Abhebern der Mutterlauge mittels einer Lanze) und sie dann zur Ringschlußreaktion in wäßriger Kalilauge oder vorzugsweise wäßriger Natronlauge zu dispergieren.

Ein Vorteil des erfindungsgemäßen Verfahrens ist darin zu sehen, daß es praktisch ohne organische Lösungsmittel durchgeführt werden kann, wobei die aufwendige Rückgewinnung der Alkohole, die beim Verfahren des Standes der Technik zur Anwendung kommen, entfällt.

Mittels des neuen Verfahrens erhält man die Phenyloxdiazolylaniline I in Form von feinteiligen Kristallen. Sie können nun in verdünnter Salzsäure mit Natriumnitrit vollständig diazotiert werden. Dies ist von besonderem Vorteil, da dadurch die große Menge an Säure, wie sie bei der Diazotierung in Schwefelsäure/Nitrosylschwefelsäure anfällt, vermieden wird.

Durch Kupplungsreaktion der in verdünnter Salzsäure hergestellten Diazoniumverbindung mit einer geeigneten Kupplungskomponente (z.B. 3-Cyano-6-hydroxy-4-methylpyrid-2-on) erhält man in guter Ausbeute Pigmente, die die gleichen anwendungstechnischen Eigenschaften aufweisen, wie jene, die nach dem in der DE-A-2 457 687 beschriebene Verfahren hergestellt wurden.

Weiterhin günstig ist, daß nunmehr eine solchermaßen erhaltene Pigmentsuspension mit Natronlauge alkalisch gestellt und das Pigment in an sich bekannter Weise mit Dispergierhilfsmitteln, z.B. mit natürlichen oder synthetischen Harzen, im Eintopf belegt werden kann. Bei dem im Stand der Technik beschriebenen Verfahren muß das Pigment vor der Harzbelegung nämlich isoliert und erneut in Wasser dispergiert werden, da sonst wegen des hohen Salzanfalls bei der Neutralisation kein Durchmischen des Reaktionsansatzes möglich ist.

Die folgenden Beispiele sollen die Erfindung weiter erläutern.

### Beispiel 1

120 g Benzonitril und 7,5 g eines anionischen Tensids (Natriumsalz von sulfoniertem Ölsäuredibutylamid) wurden eine Stunde bei Raumtemperatur in 375 ml Wasser dispergiert. Nach Zugabe von 98 g Hydroxylamnoniumsulfat wurden portionsweise 67,5 g Natriumcarbonat zugegeben. Es wurde auf 90°C erwärmt und fünf Stunden bei dieser Temperatur gerührt. Anschließend kühlte man auf 60°C ab und gab bei dieser Temperatur portionsweise 190 g Isatosäureanhydrid hinzu. Nach Erwärmen auf 90°C wurde das Reaktionsgemisch, das die ringoffene Verbindung enthält, mit 50 gew.%iger Natronlauge auf einen pH-Wert von 10,5 bis 11,0 gestellt und eine Stunde bei dieser Temperatur nachgerührt. Man saugte den Niederschlag ab, wusch mit Wasser, trocknete und erhielt 222 g 2-(3-Phenyl-1,2,4-oxdiazol-5-yl)anilin in einer feinteiligen Kristallform.

### Beispiel 2

Man verfuhr analog Beispiel 1, jedoch wurde nach Zugabe von 190 g Isatosäureanhydrid auf 90°C erwärmt und eine Stunde bei dieser Temperatur gerührt. Man verdünnte dann mit kaltem Wasser auf ein Volumen von 3 l und heberte die Mutterlauge nach Absetzen des Produktes mit einer Lanze ab. Diese Prozedur wurde zweimal wiederholt. Nach erneuter Zugabe von Wasser wurde der Reaktionsansatz unter Rühren auf 90°C erhitzt und mit 50 gew. %iger Natronlauge auf einen pH-Wert von 10,5 bis 11 gestellt. Nach zwei Stunden Rühren bei 90°C wurde das Zielprodukt abgesaugt, gewaschen und getrocknet. Man erhielt 223 g 2-(3-Phenyl-1,2,4-oxdiazoly-5-yl)anilin in einer feinteiligen Kristallform.

### Beispiel 3

Man verfuhr analog Beispiel 1, jedoch wurde die ringoffene Verbindung abgesaugt, gewaschen und erneut in 2000 ml Wasser dispergiert. Man erwärmte auf 90°C, stellte mit 50 gew.%iger Natronlauge auf einen pH-Wert von 10,5 bis 11 und rührte zwei Stunden bei dieser Temperatur nach. Nach Absaugen, Waschen und Trocknen erhielt man 214 g 2-(3-Phenyl-1,2,4-oxdiazol-5-yl)anilin in einer feinteiligen Kristallform.

### Beispiel 4

48 g Benzonitril und 3 g eines anionischen Tensids (Natriumsalz von sulfoniertem Ölsäuredibutylamid) wurden in 150 ml Wasser eine Stunde bei Raumtemperatur dispergiert. Nach Zugabe von 39,2 g Hydroxylammoniumsulfat wurden portionsweise 27 g Natriumcarbonat eingetragen. Es wurde auf 90°C erwärmt, fünf Stunden bei dieser Temperatur gerührt und anschließend auf 60°C abgekühlt. Dann gab man 112 g 5-Bromisatosäureanhydrid hinzu und erhitzte erneut auf 90°C. Nach zwei Stunden Rühren bei dieser Temperatur wurde das Produkt abgesaugt, gewaschen und erneut in 1200 ml Wasser dispergiert. Man stellte mit 50 gew.%iger Natronlauge auf einen pH-Wert von 10,5 bis 11,0, rührte eine Stunde bei dieser Temperatur nach, saugte ab und wusch mit Wasser. Nach dem Trocknen erhielt man 110 g 4-Brom-2-(3-phenyl-1,2,4-oxdiazol-5-yl)anilln in sehr feinteiliger Kristallform.

### Beispiel 5 (Vergleich)

Zu einer Lösung von 151 g Natriumcarbonat in 650 ml Wasser wurden bei Raumtemperatur nacheinander 650 ml Isobutanol, 268 g Benzonitril und portionsweise 220 g Hydroxylammoniumsulfat gegeben. Danach wurde auf 90°C erhitzt und fünf Stunden bei dieser Temperatur gerührt. Man ließ auf 30 bis 40°C abkühlen und gab bei dieser Temperatur portionsweise 392 g Isatosäureanhydrid zu. Danach heizte man auf 80°C auf und tropfte 100 g 50 gew.%ige Natronlauge zu. Dann wurde Isobutanol durch Einleiten von Dampf quantitativ abdestilliert, der grobkristalline Rückstand abgesaugt, mit Wasser gewaschen und getrocknet. Man erhielt 470 g 2-(3-Phenyl-1,2,4-oxdiazol-5-yl)anilin in einer grobkristallinen Form.

### Beispiel 6 (Anwendung als Diazokomponente)

59 g (berechnet trocken) 2-(3-Phenyl-1,2,4-oxdiazol-5-yl)anilin (aus Beispiel 3) wurden als wasserfeuchter Preßkuchen in 700 ml Wasser und 80 ml konz. Salzsäure bei 90°C dispergiert. Man ließ unter Rühren auf Raumtemperatur abkühlen und diazotierte portionsweise mit 70 g einer 23 gew.%igen Natriumnitritlösung, wobei die Temperatur mit Eis unter 10°C gehalten wurde. Nach Zerstörung des Nitritüberschusses mit Amidosulfonsäure ließ man 36,5 g 3-Cyano-6-hydroxy-4-methylpyrid-2-on in 400 ml Wasser und 15 g 50 gew.%ige Natronlauge zulaufen. Man erwärmte auf 40°C, rührte eine Stunde bei dieser Temperatur nach, saugte ab und wusch mit Wasser. Nach dem Trocknen erhielt man 95,2 g eines gelben Pigments, das die gleichen anwendungstechnischen Eigenschaften in Druckfarben aufweist wie ein Pigment, das mit dem Oxdiazolylanilin aus Vergleichsbeispiel Nr. 5 (nach der in Beispiel 1 der DE-A-2 452 687 beschriebenen Methode) hergestellt und bei 40°C formiert wurde.

## Patentansprüche

1. Verfahren zur Herstellung von Phenyloxdiazolylanilinen der Formel I 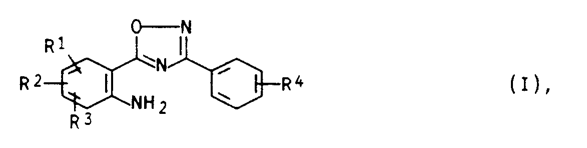 in der
R¹ Wasserstoff, Halogen, C₁-C₄-Alkyl, C₁-C₄-Alkoxy, Trifluormethyl oder Nitro,
R² Wasserstoff, Halogen oder Nitro,
R³ Wasserstoff oder Halogen und
R⁴ Wasserstoff, Halogen, C₁-C₄-Alkyl, C₁-C₄-Alkoxy oder Nitro bedeuten,
durch Umsetzung eines Benzonitrils der Formel II 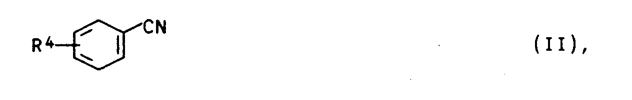 in der R⁴ die obengenannte Bedeutung besitzt, in wäßrigem Reaktionsmedium zunächst mit Hydroxylamin oder dessen Säureadditionssalzen zu einem Benzamidoxim der Formel III 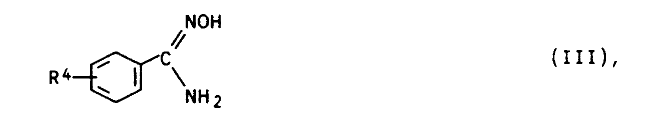 in der R⁴ die obengenannte Bedeutung besitzt, und anschließend mit einem Isatosäureanhydrid der Formel IV 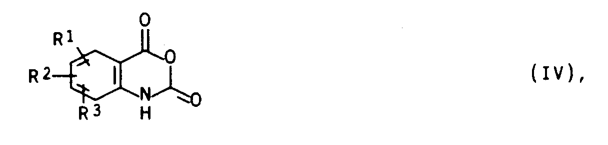 in der R¹, R² und R³ jeweils die obengenannte Bedeutung besitzen, zu einer ringoffenen Verbindung der Formel V 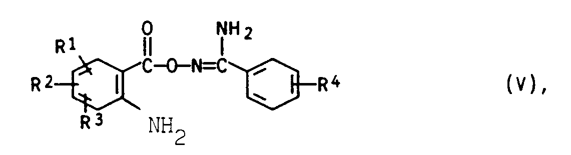 in der R¹, R², R³ und R⁴ jeweils die obengenannte Bedeutung besitzen, die dann cyclisiert wird, dadurch gekennzeichnet, daß man die Umsetzung in Gegenwart eines anionischen Tensids vornimmt.

2. Verfahren gemäß Anspruch 1, dadurch gekennzeichnet, daß man die Umsetzung mit einem Benzonitril der Formel II, in der R⁴ Wasserstoff bedeutet, und mit einem Isatosäureanhydrid der Formel IV, in der R¹ Wasserstoff, Chlor oder Brom, R² Wasserstoff, Chlor oder Brom und R³ Wasserstoff bedeuten, durchführt.

3. Verfahren gemäß Anspruch 1, dadurch gekennzeichnet, daß man die Umsetzung in Wasser durchführt, das im wesentlichen frei ist von organischen Lösungsmitteln.

## Claims

1. A process for preparing a phenyloxadiazolylaniline of the formula I 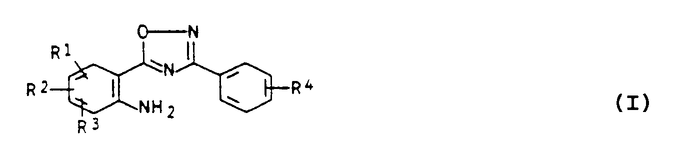 where
R¹ is hydrogen, halogen, C₁-C₄-alkyl, C₁-C₄-alkoxy, trifluoromethyl or nitro,
R² is hydrogen, halogen or nitro,
R³ is hydrogen or halogen and
R⁴ is hydrogen, halogen, C₁-C₄-alkyl, C₁-C₄alkoxy or nitro,
by reacting a benzonitrile of the formula II 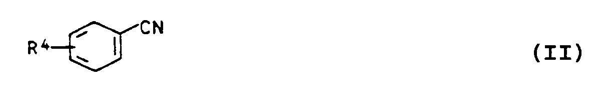 where R⁴ is as defined above, in an aqueous reaction medium first with hydroxylamine, or an acid addition salt thereof, to give a benzamideoxime of the formula III 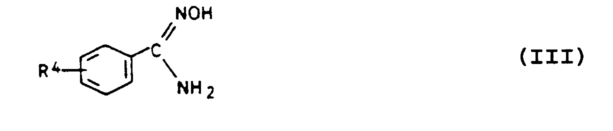 where R⁴ is as defined above, and then with an isatoic anhydride of the formula IV 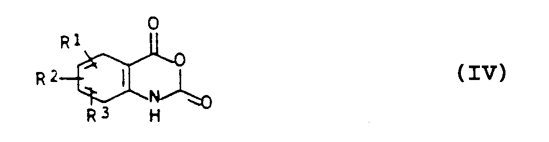 where R¹, R² and R³ are each as defined above, to give a non-cyclic compound of the formula V 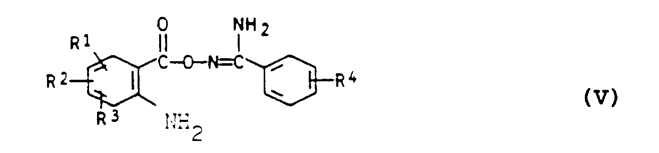 where R¹, R², R³ and R⁴ are each as defined above, and then cyclizing, which comprises carrying the reaction out in the presence of an anionic surfactant.

2. A process as claimed in claim 1, wherein the reaction is carried out with a benzonitrile of the formula II where R⁴ is hydrogen and with an isatoic anhydride of the formula IV where R¹ is hydrogen, chlorine or bromine, R² is hydrogen, chlorine or bromine and R³ is hydrogen.

3. A process as claimed in claim 1, wherein the reaction is carried out in water which is essentially free of organic solvent.

## Revendications

1. Procédé de préparation de phényloxadiazolylanilines de formule 1 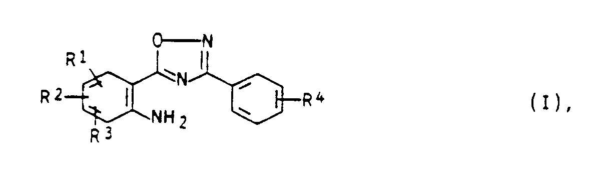 dans laquelle
R¹ représente un atome d'hydrogène, d'halogène, un groupe alkyle en C₁-C₄, alcoxy en C₁-C₄, trifluoromèthyle ou nitro,
R² représente un atome d'hydrogène, d'halogène ou un groupe nitro,
R³ représente un atome d'hydrogène au d'halogène et
R⁴ représente un atome d'hydrogène, d'halogène, un groupe alkyle en C₁-C₄, alcoxy en C₁-C₄ ou nitro,
par réaction d'un benzonitrile de formule II 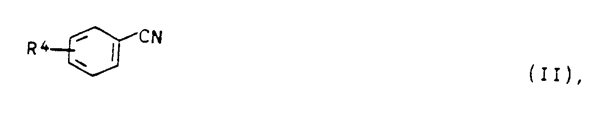 dans laquelle R⁴ a la signification indiquée ci-dessus, dans un milieu réactionnel aqueux, en premier lieu avec de l'hydroxylamine ou l'un de ses sels d'addition d'acide, en une benzamidoxime de formule III 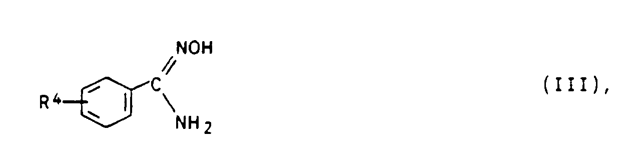 dans laquelle R⁴ a la signification indiquée ci-dessus, puis avec un anhydride d'acide isatoïque de formule IV 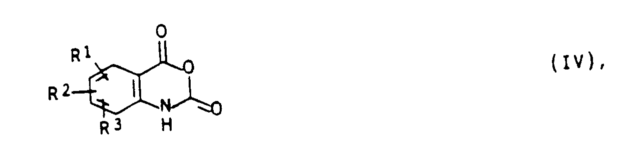 dans laquelle R¹, R² et R³ ont chaque fois la signification indiquée ci-dessus, en un composé à cycle ouvert de formule V 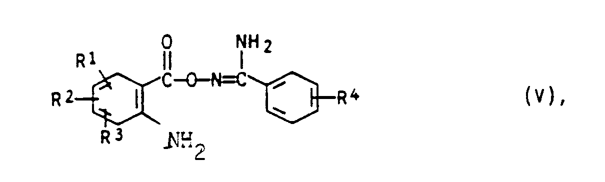 dans laquelle R¹, R², R³ et R⁴ ont chaque fois la signification indiquée ci-dessus, que l'on soumet à une cyclisation, caractérisé en ce que l'on effectue la réaction en présence d'un tensioactif anionique.

2. Procédé selon la revendication 1, caractérisé en ce que l'on effectue la réaction avec un benzonitrile de formule II, dans laquelle R⁴ signifie un atome d'hydrogène, et avec un anhydride d'acide isatoïque de formule IV, dans laquelle R¹ est un atome d'hydrogène. de chlore ou de brome, R² est un atome d'hydrogène, de chlore ou de brome et R³ est un atome d'hydrogène.

3. Procédé selon la revendication 1, caractérisé en ce que l'on effectue la réaction dans de l'eau qui est exempte pour l'essentiel de solvants organiques.
